Europäisches Patentamt

**European Patent Office**

**Office européen des brevets**

(11) Publication number: **0 270 822**
**A1**

# EUROPEAN PATENT APPLICATION

(21) Application number: **87115985.1**

(51) Int. Cl.⁴: **C12N 15/00 , A01H 1/00**

(22) Date of filing: **30.10.87**

The applicant has filed a statement in accordance with Rule 28 (4) EPC (issue of a sample only to an expert). Accession number(s) of the deposit(s): DSM 3893, DSM 4286, DSM 4287.

(30) Priority: **31.10.86 EP 86115154**

(43) Date of publication of application:
**15.06.88 Bulletin 88/24**

(84) Designated Contracting States:
**AT BE CH DE ES FR GB GR IT LI LU NL SE**

(71) Applicant: **Max-Planck-Gesellschaft zur Förderung der Wissenschaften e.V.**
**Bunsenstrasse 10**
**D-3400 Göttingen(DE)**

(72) Inventor: **Leemans, Jan, Dr.**
**Plant Genetic Systems N.V. J.Plateaustraat 22**
**B-9000 Gent(BE)**
Inventor: **Deblaere, Rolf, Dr.**
**Plant Genetic Systems N.V. J.Plateaustraat 22**
**B-9000 Gent(BE)**

(74) Representative: **Vossius & Partner**
**Siebertstrasse 4 P.O. Box 86 07 67**
**D-8000 München 86(DE)**

(54) **Stable binary agrobacterium vectors and their use.**

(57) Binary vectors for the introduction of DNA into plant cells are presented. They contain the origin of replication of the Pseudomonas plasmid pVS1 and and a T-DNA region consisting of the border sequences of the $T_L$-DNA of the pTiB6S3 plasmid and a nos-NPTII chimaeric gene to select kanamycin resistance in plant cells. The vectors replicate completely stably in Agrobacterium . Transformation of tobacco protoplasts using the binary vector is equally efficient as for previously described vector systems. The transferred T-DNA is delineated by the border sequences and in general only one or few copies of the T-DNA become integrated into the plant genome.

EP 0 270 822 A1

0 270 822

Max-Planck-Gesellschaft zur Förderung der
Wissenschaften e.V.

87115985-1

"Stable Binary Agrobacterium Vectors and their Use"

Transfer of foreign DNA into plant genomes is most often performed using plasmid vector systems derived from the tumor-inducing plasmids of Agrobacterium tumefaciens. T-DNA, a fragment of these plasmids, is transferred into the plant chromosomes during the naturally occuring process of crown gall tumor formation (for a review see Gheysen et al, 1985). The host range of Agrobacterium is very wide and recently, transformation of Liliaceae by Agrobacterium has been reported (Hernalsteens et al, 1984). This contradicted the classical observations, which indicated that the applicability of the Agrobacterium system would be limited to dicotylodoneous plants. However, Agrobacterium -mediated transformation of Gramineae, including the important cereal crops, has not yet been achieved. Other emerging techniques to introduce foreign genes into plants are the uptake of naked DNA by protoplasts after polyethylene glycol treatment, electroporation or a combination of both. Although these methods can be applied to both monocot and dicot species, the use of protoplasts very often limits the possibilities to regenerate normal plants after transformation.

At present, a wide variety of Agrobacterium vector systems has been developed. These allow easy cloning of foreign genes and they are transferred in a single step from E. coli into A.tumefaciens. Most important, transformation can be obtained after infection of wounded plant parts, such as stems, leaf segments, tuber slices or root slices. Two observations allowed the design of the presently used Agrobacterium vectors: first the T-DNA genes responsible for phytohormone - independence of crown gall cells are not required for T-DNA transfer and integration. Second, the T-DNA borders, including the directly repeated sequences of 25 bp were shown to be the only essential cis-acting sequences involved in T-DNA transfer.

One type of Ti-plasmid derived vectors are intermediate or shuttle vectors which recombine through homologous recombination with a receptor Ti-plasmid (Zambryski et al, 1983). A second type are binary vector systems (Bevan et al, 1984) in which T-DNA is carried by a vector molecule, capable of autonomous replication in Agrobacterium. Ti-plasmid functions required for T-DNA transfer are complemented in trans by the vir genes, present on a Ti-plasmid derivative (Hoekema et al, 1983). Agrobacterium strains are equally easy to construct using recombination or binary vectors, but the use of binary vectors omits the need to verify the structure of the recombinant Ti-plasmid by Southern blot and hybridization analysis. Most important, it is hoped that complete libraries of plant genomic DNA, constructed in cosmid derivatives of binary vectors, can be transferred 'en masse' into plant cells. This could allow the complementation of biochemical mutants or selection for the expression of dominant genes. In the resulting plant cell lines the gene of interest would be easily recognized due to the cotransferred T-DNA sequences. This would enable the cloning of this gene. Such experiment would involve a large number of clones and the stability of the binary vectors will determine whether complete libraries can be successfully transferred. The binary vectors presently described are based on broad host range plasmids, such as pRK252 (Bevan, 1984) and pTJS75 (An et al., 1985), which are derived from the P-type plasmid RK2, or contain an origin of replication from an Agrobacterium plasmid (Siemoens et al., 1986)

However, a major consideration in the use of these replicons is their considerable instability under non-selective conditions (Ditta et al., 1985). Therefore there existed a need for other plasmids which might lead to more stable Agrobacterium replicons.

It is the object of the present invention to provide novel binary vectors for the introduction of foreign DNA into plant cells which vectors allow easy cloning, are mobilizable at high frequency to Agrobacterium where they replicate autonomously and which display improved stability under non-selective conditions.

This object is achieved on the basis of the surprising finding, that binary vectors containing the origin of replication of the Pseudomonas plasmid pVS1 show the above mentioned desirable characteristics.

pVS1 is a 30kb Pseudomonas aeruginosa plasmid that confers resistance to mercury ions and sulfonamide (Stanisich et al., 1977). pVS1 can be efficiently mobilized into Agrobacterium by the P plasmid RP1 (Itoh et al., 1984).
The subject matter of the invention are novel binary vectors for the introduction of foreign DNA into plant cells which comprise, the origin of replication of the Pseudomonas plasmid pVS1, an origin for replication in E. coli, and a T-DNA region of the $T_L$-DNA of the octopine-type Ti-plasmid provided with different adapter fragments, transcription regulatory signals and flanked by the right and left border sequence.

In accordance with the present invention the construction of a novel binary vector system for <u>Agrobacterium</u>-mediated transformation of plant cells is achieved. Binary vectors provide a practical advantage over "classical" vector systems in that they do not require to integrate the vector in a resident Ti-plasmid through homologous recombination. The pVS1-derived vectors of the invention have following useful properties:

- They are sufficiently small to allow easy cloning (ca 10 kb).

- They are mobilizable at high frequency to <u>Agrobacterium</u>.

- The origin of replication (4 kb fragment) is derived from the 30kb pVS1 plasmid and hence is able to replicate recombinant plasmids of relatively large size.

- T-DNA transfer using the binary vectors of the invention is equally efficient as with recombinational vectors. Hence, <u>trans</u> complemetation of the <u>vir</u>-gene products appears fully effective.

- If binary vectors are to be used to perform shotgun cloning experiments, the pVS1 derived vectors will prove very useful since they do not exhibit any instability, neither under normal growth conditions, nor during plant transformation.

The binary vectors of the present invention are a useful tool for the construction of gene libraries, i.e. of genomic libraries or of cDNA libraries. Preferred host organisms are <u>Agrobacterium.</u>

From the molecular analysis of the T-DNA stucture in 6 transformed cell lines it is clear that:

1) T-DNA borders are recognized to delineate most of the T-DNA inserts.

2) The copy number of the inserts is not different from those obtained using recombinational vectors. The invention will now be described in more detail with reference to the accompanying drawing.

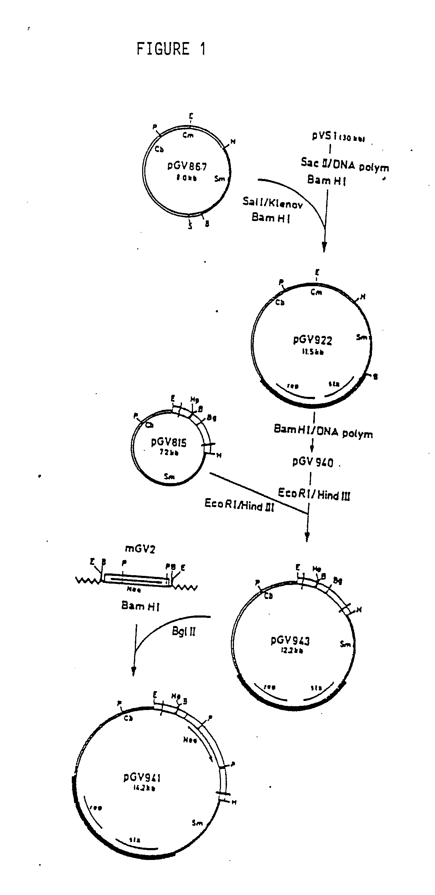

Figure 1. Construction of the pVS1 derived binary vector pGV941.
A 3.8kb BamHI/SacII fragment of pVS1, carrying the stability and replication functions was cloned into the BamHI/SalI site of pGV867, producing pGV922. SacII and SalI sticky ends were converted to blunt end by DNA polymerase treatment prior to cloning. The unique BamHI site of pGV922 was eliminated by filling in the sticky ends and religating the plasmid, resulting in pGV940. A 1.87kb EcoRI/HindIII fragment, isolated from pGV815 (Deblaere et al., 1985) and containing the octopine $T_L$-DNA border sequences, was cloned into the EcoRI/HindIII sites of pGV940, producing pGV943. The chimaeric NPTII gene from mGV2 was isolated as a 2.4kb BamHI fragment and inserted into the unique BglII site of pGV943. The resulting plasmid pGV941 contains a unique BamHI and HpaI site for cloning. This construct was deposited with DSM Gottingen, F.R.G. and received the number DSM 3893.

Abbreviations: Cb: carbenicillin resistance; Cm: chloramphenicol resistance; Neo: neomycin resistance; Sm: streptomycin and spectinomycin resistance; B: BamHI; Bg: BglII; E: EcoRI; h: HindIII; Hp: HpaI; P: PstI; S: SalI; rep: replication function; sta: stability function.

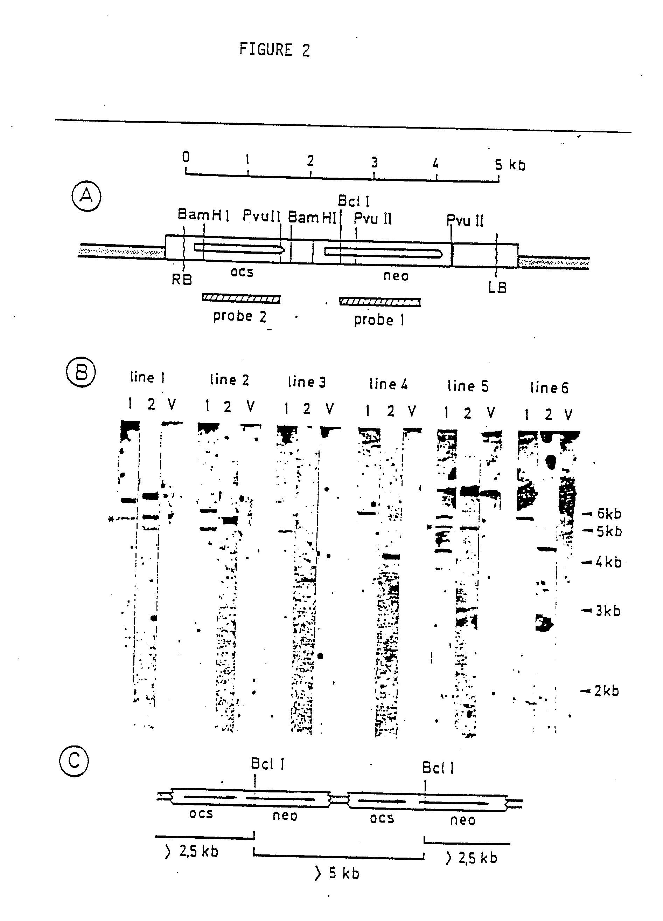

Figure 2. Physical analysis of the T-DNA structure in pGV944 transformed cell lines.

A.      The restriction map of the T-region of pGV944. The dotted areas represent the vector part of pGV944. The wavy lines indicate the $T_L$-DNA border sequences. The fragments used as probe are indicated.

B.      Hybridization pattern of 6 independent cell lines transformed with C58C1Rif$^R$(pGV2260)(pGV944). 10 ug of total plant DNA was digested with BclI, separated by agarose gel electrophoresis, blotted onto nylon filter and hybridized to radioactively labelled probes :

Probe 1:  the 1.0 kb BclI/Sma fragment from pKC7 (Rao and Rogers, 1979) covering the Km$^R$ structural gene;

Probe 2:  the 1.4 kb BamHI/PvuII fragment, containing the ocs structural gene and isolated from mGV1 (Deblaere et al., 1985);

Probe V:  the plasmid pGV922.

C   .   Representation of a tandem duplication configuration of the
        pGV944 T-DNA; the size of the expected BclI fragments is
        indicated. Bands corresponding to a possible junction fragment
        are indicated in B by an asterisk.

Figure 3.

A.   Construction of the binary vector pGSC1701A2
     The 800 bp PvuII-NdeI fragment from pJE21 was isolated and
     introduced into pGV943. The latter was partially digested with NdeI
     and subsequently with EcoRI. A partial NdeI digest was required
     since an additional NdeI site is present in the pVS1 origin of
     replication. The EcoRI sticky ends could be ligated with the PvuII
     blunt ends after Klenow DNA polymerase treatment. The resulting
     plasmid pGV943A was subsequently digested with SacI and NdeI, and
     the fragment containing the origin of replication (ori*) was
     inserted in pGSC1701. The latter plasmid was digested with the same
     enzymes. This construction yielded the binary vector pGSC1701A2. This
     construct was deposited with DSM Göttingen, F.R.G. and received the
     number DSM 4286).

B.   Construction of the expression vector pGSFR761A.
     Three marker genes under control of a constitutive promoter are
     inserted between the T-DNA borders of pGSC1701A2. A BamHI-HindIII
     fragment from pGSFR161 was inserted in the polylinker region of
     pGSC1701A2 which was linearized with BamHI and HindIII. This
     fragment contains the bar and neo gene hooked up to the divergently
     transcribing T-DNA TR promoters. This yielded pGSFR760A.
     Subsequently a chimeric construct comprising the hyg gene fused to
     the 35S promoter was isolated as a BglII fragment, blunt ended with
     Klenow DNA polymerase and cloned in the filled HindIII site of
     pGSFR760A. This yielded pGSFR761A. This contruct was deposited with
     DSM Göttingen, F.R.G. and received the number DSM 4287.

All plasmid constructs are schematically represented; their size is indicated in kb under the name of the plasmid. Open boxes represent relevant features of the plasmids. Abbrivations: Amp: ampicillin resistance; Sm: streptomycin/spectinomycin resistance; RB: right border; LB: left border; PVS1: restriction fragment containing the pVS1 origin of replication; 3'7; 3'ocs, 3'nos: termination and polyadenylation signals of T-DNA gene 7, octopine synthase and nopaline synthase respectively; TR2', TR1': divergently transcribing promoters from the TR T-DNA gene 1' and 2'; bar: bialaphos resistance gene; nptII: neomycin phosphotransferase II gene; hyg: hygromycin phosphotransferase gene; T-DNA octopine T-DNA sequences left between the border fragments.

## Materials and methods

## Bacterial strains and plasmids.

E. coli strain K514 (Colson et al, 1965) was used throughout the recombinant DNA work. Agrobacterium strain C58C1Rif$^R$ (pGV2260) was used to provide in trans the vir-functions for the binary vectors (Deblaere et al, 1985).

pGV867 is a pBR325 derivative carrying a 2.25kb HindIII/BamHI fragment which encodes streptomycin and spectinomycin resistance. pGV815 and pGV825 are intermediate vectors, containing an avirulent T-DNA (Deblaere et al,1985).

pGV2486 is a cointegrate between pGV2260 and pGV891. pGV891 is similar to pGV833 (Deblaere et al., 1985): the chimaeric NPTII gene was isolated as a BamHI fragment from mGV2 - derived from M13mp7 with a Pnos-neo-3'ocs cassette - and inserted in the unique BglII site of pGV825. The ocs gene was subsequently inserted as a BamHI fragment as previously described for the construction of pGV833.

## DNA techniques

Recombinant DNA techniques were performed as described (Maniatis et al., 1982).

## Bacterial conjugations.

Binary vectors were mobilized from E. coli to A. tumefaciens in a triparental mating using helper plasmid pRK2013 (Ditta et al, 1980). 0.1 ml of exponentially growing cultures of donor, acceptor and helper strain were plated on a LB agar plate, and incubated overnight at 28°C. The mobilization mixture was recovered in 2 ml $10^{-2}$ M MgSO$_4$ and serial dilutions were plated on selective medium. Transconjugants were selected on minimal medium containing streptomycin (1000 ug/ml) and spectinomycin (300 ug/ml). The skilled practitioner knows other strains of E. coli and of Agrobacterium which provide in trans the vir functions as well as other helper plasmids which can equally be used for carrying out the present invention.

## Plant transformations.

Agrobacterium strains carrying the binary vector were tested in a protoplast cocultivation system. Mesophyll protoplasts were isolated from Nicotiana tabacum cv. Petit Havana SR1 (Maliga et al, 1973) and cocultivated as described by Marton et al.(1979). Transformed plant cells were selected for kanamycin resistance as described by De Block et al.(1984). Octopine synthase activity in transformed cell lines was assayed according to Leemans et al (1981).

## T-DNA analysis.

Total plant DNA was isolated as described previously (Dellaporta et al, 1984). Digested plant DNA was transferred to a Hybond N nylon membrane (Amersham), using the classical Southern blot procedure. DNA-DNA hybridization was performed according to the suppliers instructions.

## Example


A) <u>Construction of binary vectors.</u>


The stability and replication functions of the <u>Pseudomonas</u> plasmid pVS1 are carried on a 3.8kb BamHI/SacII restriction fragment (Itoh et al., 1984). This fragment was used in the construction of plasmid pGV943 (see figure 1). This binary vector contains a segment of pBR325, providing carbenicillin resistance and replication functions. The latter are necessary since pVS1 does not replicate in <u>E. coli</u>. pGV943 carries a bacterial streptomycin-spectinomycin resistance marker to facilitate selection in <u>Agrobacterium</u>. A 1.87kb T-DNA fragment, recloned from the cointegration vector pGV815 (Deblaere et al., 1985) and containing the left and right border sequences of the octopine $T_L$-DNA, was included. This avirulent T-DNA contains unique restriction sites for BamHI, BglII and HpaI cloning.

To be used as a control, the binary vector pGV912 was constructed, based on the broad host range replicon pRK404 (Ditta et al., 1985) and containing an identical T-DNA as pGV943. A 2.8kb PvuII fragment, carrying an avirulent T-DNA and the streptomycin-spectinomycin resistance gene, was isolated from pGV825 (Deblaere et al., 1985) and cloned into the SmaI site of pRK404, producing pGV912. The stability and plant transforming properties of pGV912 are compared to those of pGV943.

To successfully apply these binary vectors in plant transformation experiments, a chimaeric neomycin phosphotransferase II (NPTII) gene was included as dominant selectable marker gene (Herrera-Estrella et al., 1983; Bevan et al., 1983; Fraley et al. 1983). The chimaeric NPTII gene was isolated from pLGVneo1103 (Lörz et al., 1985) as a 2.3kb EcoRI/SalI fragment. It was converted to blunt-end by treatment with the Klenow fragment of the <u>E. coli</u> DNA polymerase and subcloned in the HincII site of M13mp7 (Vieira and Messing, 1982), producing mGV2. The NPTII gene was recloned as a BamHI fragment and inserted in the unique BglII site of either pGV912 and pGV943, resulting in pGV915 and pGV941 respectively.

The construct pGV941 was deposited with the Deutsche Sammlung für Mikroorganismen (DSM) and reveived the deposition no. 3893.

In a model experiment, the octopine synthase (ocs) gene was isolated from mGV1 as a 1.4kb BamHI fragment and inserted in the unique BamHI site of pGV915 and pGV941 as described previously for the cointegration vector (Deblaere et al., 1985), producing pGV939 and pGV944 respectively. The ocs gene was chosen to detect gene expression of additionally introduced genes in the T-DNA, because ocs activity can easily be detected in transformed plant cells (Leemans et al., 1981).

B) Stability of binary vectors in Agrobacterium.

The binary vectors pGV939 and pGV944 were mobilized from E. coli to Agrobacterium strain C58ClRif^R(pGV2260) in a triparental mating using HB101(pRK2013) as a helper. Streptomycin-spectinomycin resistant transconjugants arose at a frequency of 1% in both crosses. The stability of the vector plasmids was determined in cultures of C58ClRif^R(pGV2260)(pGV939) and C58ClRif^R(pGV2260)(pGV944), grown overnight in selective LB medium, containing streptomycin (300ug/ml) and spectinomycin (100ug/ml). Bacteria were plated on non-selective LB agar medium and resulting colonies were screened for spectinomycin resistance. Furthermore, the cultures were diluted and grown for 7, 14 and 21 generations in non-selective liquid medium before plating on non-selective LB plates. Again, colonies were screened for their spectinomycin resistance. Table 1 shows that pVS1 and its derived plasmids are completely stable in Agrobacterium, whereas pRK404 and its derivative pGV939 are lost at high frequency during non-selective growth.

Table 1

Plasmid stability in Agrobacterium strain C58ClRif^R

| Number of generations | Plasmids | | | | |
| | pVS1 | pGV922 | pGV944 | pGV939 | pRK404 |
| --- | --- | --- | --- | --- | --- |
| 0 | >99 | >99 | >99 | 76 | 68 |
| 7 | >99 | >99 | >99 | 74 | 60 |
| 14 | >99 | >99 | >99 | 56 | 54 |
| 21 | >99 | >99 | >99 | 44 | 45 |

The numbers indicate the percentage of antibiotic resistant bacteria after screening of the non-selectively grown cultures. pVS1 was screened for sulfathiazole resistance (250ug/ml), pRK404 for tetracycline resistance (5ug/ml) and the other plasmids for spectinomycin resistance (100ug/ml).

The loss of both vectors was also measured under conditions of a classical tobacco protoplast cocultivation experiment. 10ul of a selectively grown bacterial culture was added per 1ml of protoplast suspension. Every 24 hours a sample was taken from the medium and the bacteria were screened for antibiotic resistance. Again the pVS1 derived pGV944 proved completely stable ( >99% spectinomycin resistance), whereas 24 hours after infection, only 68% of the Agrobacterium cells still contained pGV939.

C) Plant cell transformations.

Mesophyll protoplasts were isolated from Nicotiana tabacum cv Petit Havana SR1 and cocultivated with the following strains: C58ClRif$^R$(pGV2260)(pGV939), C58ClRif$^R$(pGV2260)(pGV944) and C58ClRif$^R$(pGV2486). pGV2486 contains an identical T-DNA as the binary vectors, but in a cointegration vector. Transformed plant cells were selected for kanamycin resistance (50ug/ml) on solid medium as described by De Block et al.(1984). Kanamycin resistant calli were obtained at a frequency of 4% for the cointegration vector pGV2486 and 1 and 5% for the binary vectors pGV939 and pGV944 respectively.
Kanamycin resistant calli were further cultured on solid medium containing 50ug/ml kanamycin and screened for octopine synthase activity. A 100% correlation between kanamycin resistance and octopine synthesis in clones transformed with C58ClRif$^R$(pGV2260)(pGV944) was observed. In 24 clones transformed with the pRK404 derived pGV939, aswell as with pGV2486, 17 showed variable levels of octopine synthase activity and in 7 calli no octopine production was detected.

D) <u>T-DNA structure in transformed cell lines.</u>

Total plant DNA from 6 kanamycin resistant pGV944 transformed cell lines was analysed by Southern blotting to determine T-DNA copy number and structure. Hybridization to probes derived from the left and right part of the T-DNA allows one to accurately determine the T-DNA copy number. The hybridization pattern reveals that lines 3, 4 and 6 have only a single T-DNA insert, while multiple inserts are present in lines 1 (three copies), 2 (two copies) and 5 (>four copies) (see figure 2).

To investigate whether the T-DNA border sequences have been recognized to delineate the inserted T-DNA, pGV922 was used as probe. It contains all vector sequences present in pGV944, but lacks the T-DNA. Only one of the T-DNA copies in cell line 5 hybridized to vector sequences.

Cell line 1 contains a 5 kb fragment, which hybridizes to both the left and right side of the T-DNA. This is characteristic for a tandem organization of the multiple T-DNA inserts. A similar junction fragment is also observed in line 5, but not in line 2.

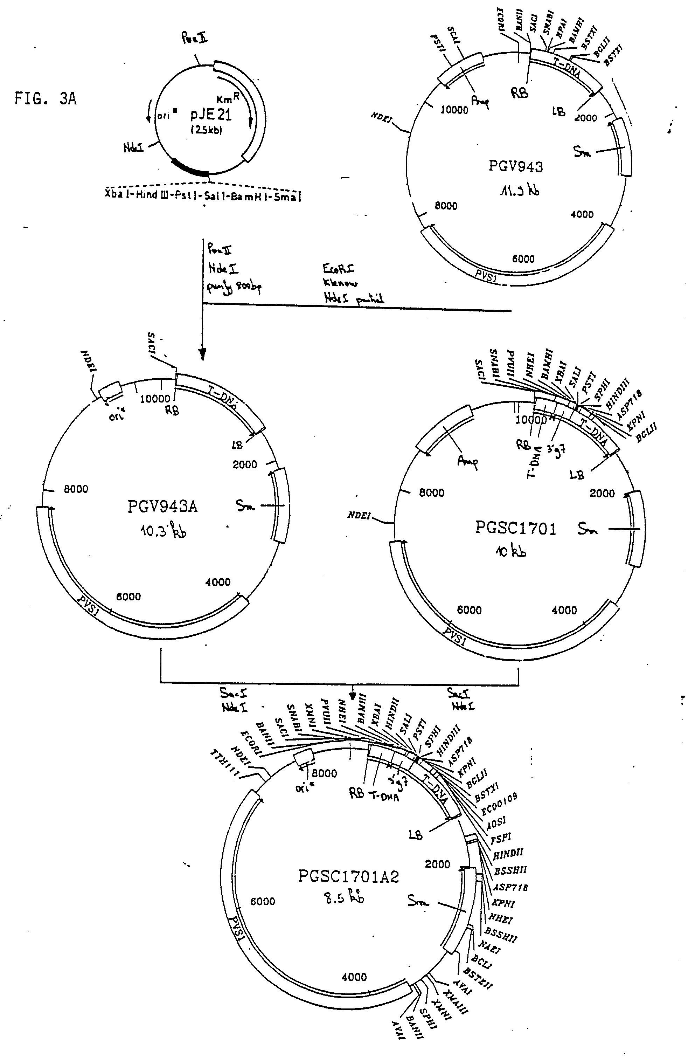

E)    Further binary vector constructs and applications.
   -  Classical Agrobacterium-mediated transformation procedures for
      plants consists of two major steps: first, plant tissue is
      infected with a culture of the Agrobacterium strain which
      carries the plasmid vector in question; secondly, the
      agrobacteria are removed using antibiotics in order to avoid
      contaminated plants. Usually the antibiotic cefotaxime is used.
      Due to the instability of the latter, plant explants have to be
      frequently subcultured during the regeneration phase, which
      might be disadvantageous in some transformation procedures. As
      alternative, the more stable carbenicillin antibiotic can be
      used in the medium. To this end, the beta-lactamase gene which
      confers resistance to ampicillin and carbenicillin was deleted
      from the binary vector pGV943 (see Fig. 1). As shown in Fig. 3A,
      a PvuII-NdeI fragment from pJE21 (Botterman, 1986) carrying a
      mutagenized ColE1 origin of replication was substituted for the
      EcoRI-NdeI fragment, which contains the beta-lactamase gene and
      a ColE1 origin of replication, of pGV943. This yielded pGV943A.
      This plasmid was perfectly stable in Escherichia coli and in
      Agrobacterium in non-selective conditions on selective LB-agar plates
      containing streptomycin and spectomycin Sm (20µg/ml) & Sp(50µg/ml) for
      E. coli and Sm (100 ug/ml) & Sp (300 ug/ml) for Agrobacterium .

      A more versatile vector was constructed by inserting a
      polylinker region and a fragment encoding termination and
      polyadenylation signals. A SacI-NdeI fragment from pGSC1701 was
      substituted for into pGV943A yielding pGSC1701A2. pGSC1701 is a
      derivative from pGV941 which contains a polylinker from pUC18
      (Yanish-Perron et al., 1985) and the 3'end of the octopine T-DNA
      gene 7 (Velten and Schell, 1985). Chimeric genes comprising transcription initiation signals fused to a gene of interest can be easily introduced in the polylinker in front of the termination signals.

pGSC1701A2 has been the basic plasmid vector for different chimeric constructs used for plant transformation. This plasmid can be mobilised into C58C1Rif$^R$ (pMP90) (Koncz and Schell, 1987) or in C58C1Rif$^R$ (pGV2275) (Simoens et al., 1986). pMP90 and pGV2275 are Ti-plasmids which provide the vir functions in trans and which contain no resistance gene for carbenicillin.

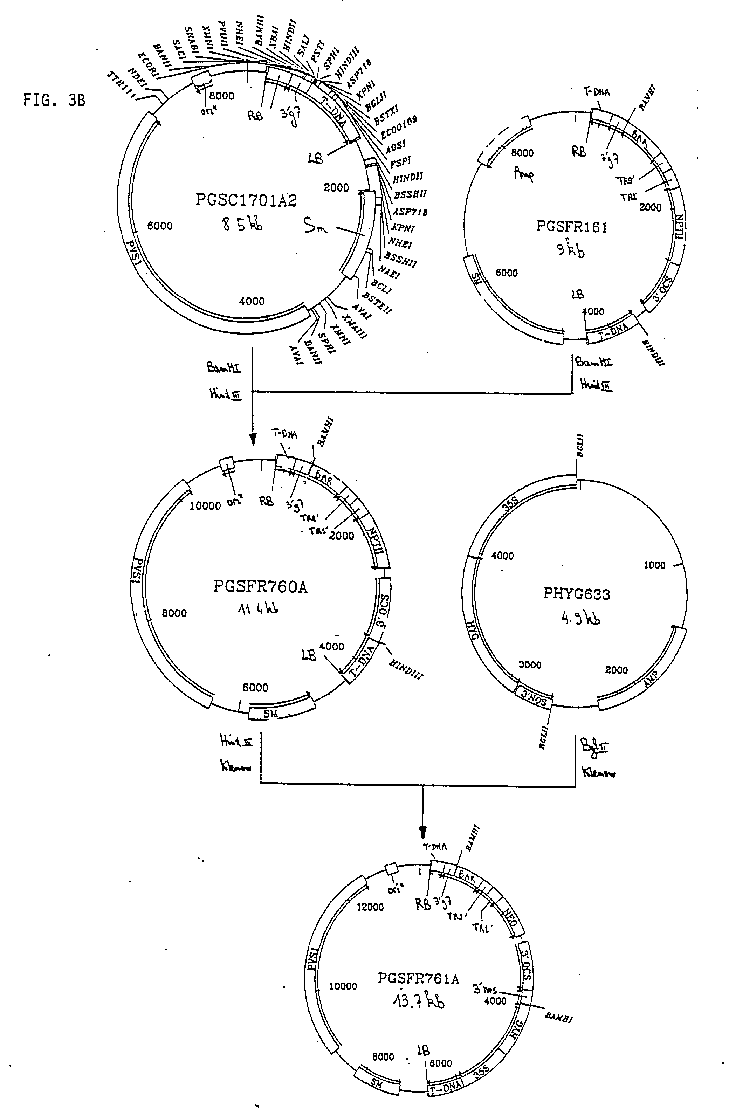

As exemplified in Fig. 3B three expression units were introduced between T-DNA borders of this plasmid. The BamHI-HindIII fragment from pGSFR161 carries the neo and bar gene hooked up to the dual T-DNA promoter fragment (Velten and Schell, 1985). Those genes encode neomycin phosphotransferase II and phosphinothricin acetyltransferase respectively and confer resistance towards kanamycin and phosphinothricin respectively. The use of both genes in plant transformation have been described (Herrera-Estrella et al., 1983; De Block et al., 1987). The BglII fragment from pHyg633 contains the hygromycin phosphotransferase gene (Van den Elzen et al., 1985) under control of the cauliflower mosaic virus 35S promoter (Odell et al., 1985). This gene confers resistance to hygromycin B. The final construct pGSFR761A contains three marker genes enclosed between the T-DNA borders. They are hooked up to different constitutively active promoters and are provided with different termination and polyadenylation signals: 3'end of T-DNA gene 7, 3'end of nopaline synthase and 3'end of octopine synthase. All marker genes can be independently selected for in transformed plant cells. Hence, this vector is ideal to evaluate and compare different selection marker in plant transformation experiments, and allows flexibility in use of antibiotics to remove the agrobacteria. Described vector has been applied with success for the transformation of different plant species.

References.


An, G., Watson, B.P., Stachel, S., Gordon, M.P. and Nester, E.W. (1985)
    EMBO J. 4, 277-284

Bevan, M.W. (1984) Nucl. Acid Res. 12, 8711-8721

Bevan, M.W., Flavel, R.B. and Chilton, M.-D. (1983) Nature (London)
    304, 184-187

Colson, C., Glover, S.W., Symonds, N. and Stacey, K.A. (1965) Genetics
    52, 1043-1050

Deblaere, R., Bytebier, B., De Greve, H., De Boeck, F., Schell, J., Van
    Montagu, M. and Leemans, J. (1985) Nucl. Acid Res. 13, 4777-4788

De Block, M., Herrera-Esterella, L., Van Montagu, M., Schell, J. and
    Zambryski, P. (1984) EMBO J. 3, 1681-1689

Dellaporta, S.J. (1983) Plant Mol. Biol. Reporter 1, 19-21

Ditta, G., Stanfield, S., Corbin, D. and Helinski, D.R. (1980) Proc.
    Natl. Acad. Sci. USA 77, 7347-7351

Ditta, G., Schmidhauser, T., Yakobson, E., Lu, P., Liang, X.-W.,
    Finlay, D.R., Guiney, D. and Helinski, D.R. (1985) Plasmid 13,
    149-153

Fraley, R.T., Rogers, S.G., Horsch, R.B., Sanders, P.R., Flick, J.S.,
    Adams, S.P., Bittner, M.L., Brand, L.A., Fink, C.L., Fry, J.S.,
    Gallupi, G.R., Goldberg, S.B., Hoffman, N.L. and Woo, S.C. (1983)
    Proc. Natl. Acad. Sci. USA 80, 4803-4807

Gheysen, G., Dhaese, P., Van Montagu, M. and Schell, J. (1985) in Genetic
    flux in plants, Hohn,B. and Dennis,E.S. (eds) (Advances in plant
    gene research, vol.2) 11-47, Wien, Springer Verlag

Hernalsteens, J.-P., Thia-Toong, L., Schell, J and Van Montagu, M. (1985)
    EMBO J. 3, 3039-3041

Herrera-Esterella, L., De Block, M., Messens, E., Hernalsteens, J.-P.,
    Van Montagu, M. and Schell, J. (1983) EMBO J. 2, 987-995

Hoekema, A., Pirsch, P.R., Hooykaas, P.J.J. and Schilperoort, R.A. (1983)
    Nature (London) 303, 179-181

Itoh, Y., Watson, J.M., Haas, D. and Leisinger, T. (1984) Plasmid 11,
    206-220

Klee, H.J., Yanofski, M.F. and Nester, E.W. (1985) <u>Bio/technology</u> 3, 637-642

Leemans, J., Shaw, C., Deblaere, R., De Greve, H., Hernalsteens, J.-P., Van Montagu, M. and Schell, J. (1981) <u>J. Mol. Appl. Genet.</u> 1, 149-164

Lörz, H., Baker, B. and Schell, J. (1985) <u>Mol. Gen. Genet.</u> 199, 178-182

Maliga, P., Breznovitz, A. and Marton, L. (1973) <u>Nature New Biol.</u> 244, 29-30

Maniatis, T., Fritsch, E.F. and Sambrook, J. (1982) Molecular Cloning : A laboratory Manual (Cold Spring Harbor, New York: Cold Spring Harbor Laboratory).

Marton, L., Wullems, G.J., Molendijk, L. and Schilperoort, R.A. (1979) <u>Nature</u> (London) 277, 129-130

Rao, R.N. and Rogers, S.G. (1979) <u>Gene</u> 7, 79-82

Stanisich, V.A., Bennett, P.M. and Richmond, M.H. (1977) <u>J. Bacteriol.</u> 129, 1227-1233

Vieira, J. and Messing, J. (1982) <u>Gene</u> 19, 259-268

Zambryski, P., Joos, H., Genetello, C., Leemans, J., Van Montagu, M. and Schell, J. (1983) <u>EMBO J.</u> 2, 2143-2150

- Simoens, C., Alliotte, T., Mendel, R., Muller, A., Schumann, J., Van Lijsebettens, M., Schell, J., Van Montagu, M. and Inzé, D. (1986). Nucl. Acids Res. 14, 8073-8090.
- Botterman, J., PhD Dissertation. State University of Ghent, 1986.
- Velten, J. and Schell, J. (1985). Nucl. Acids Res. 13, 6981-6998.
- Yanish-Perron, C., Vierra, J. and Messing, J. (1985). Gene 33, 103-119.
- Koncz, C. and Schell, J. (1987). Mol. Gen. Genet. (1986). 204, 383-396.
- Herrera-Estrella, L., De Block, M., Messens, E., Hernalsteens, J-P., Van Montagu, M. and Schell, J (1983). EMBO J. 2, 987-995.
- De Block, M., Botterman, J. Vandewiele, H., Dockx, J., Thoen, C., Gosselé, V., Movva, N., Thompson, C., Van Montagu, M. and Leemans, J. (1987). EMBO J. 9, 2513-2518.
- Van den Elzen, P., Townsend, J., Lee, K. and Bedbrook, J. (1985). Plant Mol. Biol. 5, 299-302.
- Odell, J., Nagy, F. and Chua, N.H. (1985) Nature 313, 810.

## CLAIMS

1. A binary vector for the introduction of foreign DNA into plant cells which comprises the origin of replication of the Pseudomonas plasmid pVS1 and a T-DNA region comprising the border sequences of the $T_L$-DNA of an octopine Ti-plasmid.

2. The vector of claim 1 which additionally contains a selectable marker for plant transformation, transcription regulatory signals and adaptor fragments in between the border sequences and optionally bacterial selection markers.

3. The vector of claim 2 wherein the selectable marker for plant transformation is a chimeric kanamycin resistance gene.

4. The vector of any of claims 1 to 3 which is pGV941 (DSM No. 3893).

5. The vector of any claims 1 to 4 which additionally contains a gene encoding a gene product of interest.

6. The vector of claim 5 which is pGV944.

7. The vector pGSC1701A2 (DSM No. 4286)

8. The vector pGSFR761A (DSM No. 4287).

9. A gene library which is stably replicable in Agrobacterium comprising a binary vector of any claims 1 to 4.

10. A host cell transformed with a binary vector of any of claims 1 to 8.

11. The use of the origin of replication of the Pseudomonas plasmid pVS1 for the construction of a binary vector which is stably replicable in Agrobacterium.

FIGURE 1

FIGURE 2

FIG. 3A

FIG. 3B

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 186 425 (ELI LILLY AND CO.) <br> * Page 7, lines 20-30; claim 1 * | 1,2,10 | C 12 N 15/00 <br> A 01 H 1/00 |
| Y | | 11 | |
| | --- | | |
| X | NUCLEIC ACIDS RESEARCH, vol. 14, no. 20, 24th October 1986, pages 8073-8090, IRL Press Ltd, Oxford, GB; C. SIMOENS et al.: "A binary vector for transfering genomic libraries to plants" <br> * Summary; page 8079 - page 8080, paragraph 1 * | 9 | |
| A | Idem | 1-8,10, 11 | |
| | --- | | |
| D,Y | PLASMID, vol. 11, 1984, pages 206-220, Academic Press, Inc.; Y. ITOH et al.: "Genetic and molecular characterization of the pseudomonas plasmid pVS1" <br> * Page 217, left-hand column: "Host range of pVS1" * | 11 | |
| | --- | | TECHNICAL FIELDS SEARCHED (Int. Cl.4) |
| Y | GENE, vol. 36, no. 1/2, 1985, pages 27-36, Elsevier Science Publishers, Amsterdam, NL; Y. ITOH et al.: "Cloning vectors derived from the pseudomonas plasmid pVS1" <br> * Page 35, left-hand column, paragraph 1 * | 11 | C 12 N <br> A 01 H |
| | --- | | |
| A | EP-A-0 120 516 (RIJKSUNIVERSITEIT LEIDEN) <br> * Page 12, lines 11-22; claim 4 * <br> ---        -/- | 1-11 | |

The present search report has been drawn up for all claims

| Place of search | Date of completion of the search | Examiner |
|---|---|---|
| THE HAGUE | 10-02-1988 | MADDOX A.D. |

EPO FORM 1503 03.82 (P0401)

European Patent Office

**EUROPEAN SEARCH REPORT**

Application Number

EP 87 11 5985

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | NUCLEIC ACIDS RESEARCH, vol. 12, no. 22, November 1984, pages 8711-8722, IRL Press Ltd, Oxford, GB; M. BEVAN: "Binary agrobacterium vectors for plant transformation" * Whole document * | 1-11 | |
| A | EP-A-0 198 288 (ADVANCED GENETIC SCIENCES) * Page 17, column 32 * | 1-11 | |
| A | US-A-4 605 627 (KADO) * Column 1, last paragraph - column 2, paragraph 1; column 4, paragraph 3 * | 1-11 | |
| A | WO-A-8 603 516 (BIOTECHNICA) * Page 16, lines 12-32; page 17, line 30 - page 18, line 10 * | 1-11 | |
| A | BIO/TECHNOLOGY, vol. 3, no. 7, July 1985, pages 637-642, New York, US; H.J. KLEE et al.: "Vectors for transformation of higher plants" * Whole document * | 1-11 | TECHNICAL FIELDS SEARCHED (Int. Cl.4) |
| A | EP-A-0 140 556 (AGRIGENETICS) * Examples 2-8 * | 1-11 | |
| A | A.J.M. MATZKE et al.: "A set of novel Ti plasmid-derived vectors for the production of transgenic plants", & PLANT MOL. BIOL. 1986, 7(5), 357-65 * Page 363, left-hand column, paragraph 2 * | 1-11 | |
| | -/- | | |

The present search report has been drawn up for all claims

| Place of search | Date of completion of the search | Examiner |
|---|---|---|
| THE HAGUE | 10-02-1988 | MADDOX A.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent
Office

EUROPEAN SEARCH REPORT

Application Number

EP 87 11 5985

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | NUCLEIC ACIDS RESEARCH, vol. 13, no. 13, July 1985, pages 4777-4788, IRL Press Ltd, Eynsham, Oxford, GB; R. DEBLAERE et al.: "Efficient octopine Ti plasmid-derived vectors for agrobacterium-mediated gene transfer to plants" * Whole document * | 1-11 | |
| E | EP-A-0 223 417 (LUBRIZOL) * Claims 1-3 * | 1-3,10 | |
| P,A | EP-A-0 242 246 (PLANT GENETIC SYSTEMS) * Whole document * | 7,8 | |

TECHNICAL FIELDS
SEARCHED (Int. Cl.4)

The present search report has been drawn up for all claims

| Place of search | Date of completion of the search | Examiner |
|---|---|---|
| THE HAGUE | 10-02-1988 | MADDOX A.D. |

EPO FORM 1503 03.82 (P0401)